**Europäisches Patentamt**

**European Patent Office**

**Office européen des brevets**

(11) Veröffentlichungsnummer: **0 392 304**
**A1**

(12) **EUROPÄISCHE PATENTANMELDUNG**

(21) Anmeldenummer: **90106246.3**

(22) Anmeldetag: **31.03.90**

(51) Int. Cl.5: **A61M 1/14, A61M 1/30**

(30) Priorität: **08.04.89 DE 3911587**
**08.04.89 DE 8904445 U**

(43) Veröffentlichungstag der Anmeldung:
**17.10.90 Patentblatt 90/42**

(84) Benannte Vertragsstaaten:
**AT BE CH DE DK ES FR GB GR IT LI NL SE**

(71) Anmelder: **Dialyse-Technik med. Geräte Handels GmbH**
**Einsteinstrasse 55 - 57**
**D-7505 Ettlingen(DE)**

(72) Erfinder: **Cholewa, Peter Vinzent**
**Femerlingstrasse 7**
**D-2100 Hamburg 90(DE)**

(74) Vertreter: **Lempert, Jost, Dipl.-Phys. Dr. et al**
**Patentanwälte Dipl.-Ing. Heiner Lichti**
**Dipl.-Phys. Dr. Jost Lempert Durlacher**
**Strasse 31 Postfach 410760**
**D-7500 Karlsruhe 41(DE)**

(54) **Vorrichtung und Verfahren zur Dialyse.**

(57) Die Erfindung schlägt zur Verbesserung einer Vorrichtung zur Dialyse mit einem Dialysator, mindestens einer Pumpe, Zwischengefäßen und Drucksensoren nicht nur, aber insbesondere bei der Einnadel-Dialyse vor, daß als Zwischengefäß zumindestens ein flexibler Beutel (1) zwischen zwei Andruckplatten (31,33) angeordnet ist, von denen mindestens eine beweglich und antreibbar ist und daß der Beutel an einem Drucksensor (17) anliegt.

Fig.4

Xerox Copy Centre

## Vorrichtung und Verfahren zur Dialyse

Die Erfindung betrifft eine Vorrichtung zur Dialyse, insbesondere zur Einnadel-Dialyse, mit einem Dialysator, mindestens einer Blutpumpe, Zwischengefäßen und Drucksensoren, sowie ein Verfahren zur Durchführung einer Dialyse, wobei der Druck im Blutkreislauf zumindest hinter dem Dialysator gemessen wird.

Derartige Dialyse-Vorrichtungen sind in verschiedenster Weise bekannt. Sie dienen zur Reinigung des Blutes von Patienten mit Niereninsuffizienzen. Während bei sogenannten Zweinadelsystemen, bei denen das Blut über eine Nadel von einer Arterie des Patienten abgezogen und über eine andere Nadel in eine Vene des Patienten zurückgedrückt wird, das Blut kontinuierlich durch den Dialysator gepumpt werden kann, sind Einnadeldialysatoren bekannt geworden, bei denen das Blut durch eine gemeinsame Nadel in einer ersten Phase über ein T-Stück durch einen arteriellen Zweig abgesaugt und in einer zweiten Phase über einen venösen Zweig der Einrichtung und das T-Stück durch die gleiche Nadel in die Blutbahn des Patienten zurückgedrückt wird. Letztere Vorrichtungen sind für den Patienten an sich weniger belastend, aber im Hinblick auf die Steuerung des Ablaufs, insbesondere auf das Druckverhalten nicht unproblematisch. Zur letzteren Vorrichtung wird auf die DE-C-22 36 433 sowie auf die EP-A-229 271 verwiesen.

Bei der Dialyse, insbesondere der Einnadeldialyse, aber auch der Zweinadeldialyse werden in der Regel Zwischengefäße als Blasenfänger eingesetzt, die bei der Einnadeldialyse auch als Zwischenspeicher dienen. Dies sind Gefäße, die in der Regel kostspielig sind. Es ergeben sich hier Probleme hinsichtlich der Reinigung bzw. der Kosten, wenn die Gefäße bei jeder Dialyse erneuert werden sollen. Weiterhin ist bei beiden Dialysensystemen eine Drucküberwachung vorzunehmen, die Probleme verursachen kann. So ist in der DE-C-22 36 433 eine ziemlich aufwendige Druckmeßeinrichtung mit einer an der Blasenfalle angeschlossenen Quecksilber-Manometereinrichtung vorgesehen, wobei der Druck über den Quecksilberstand in einem Manometerschenkel mittels Fotozellen abgelesen wird. Die EP-A 229 271 zeigt lediglich schematisch Zeigermanometer, die nicht näher spezifiziert sind, aber offensichtlich Manometer mit elastischem Meßglied sind. Eines der Meßgeräte wird auch zur Steuerung der Drehzahl einer Pumpe eingesetzt. Es wurde auch schon vorgeschlagen, ein flexibles Zwischengefäß vorzusehen, das direkt einen Drucksensor beaufschlagt. In allen Fällen wird eine Drucküberwachung nur hinsichtlich der unteren Druckpreßwerte vorgesehen (Lecks, Staus), bei deren Erreichen die Anlage abgeschaltet und Alarm gegeben wird. Aufgrund der stark schwankenden Durchflüsse und Drücke im Dialysator kann nachteilige Refiltration eintreten. Die EP-A-10 48 95 zeigt ein Einnadelsystem mit einer arteriellen und einer venösen Pumpe zwischen denen ein flexibler Speicherbeutel angeordnet ist. Der bei Füllung des Beutels entstehende Überdruck bewirkt ein Umschalten zwischen arterieller und venöser Pumpe, während das Rückschalten nach vorgegebenen Umläufen letzterer erfolgt.

Die DE-C-32 05 449 zeigt ein Einnadelsystem, bei dem die Blutreinigung nicht sehr effizient ist, da es mit einer diskontinuierlich wirksamen Membranpumpe arbeitet. Der Dialysator wird nämlich nur während der Rückflußphase vom Blut durchströmt. Der Druck hinter dem Filter soll dabei dadurch konstant gehalten werden, daß der Drucksensor über eine Regeleinrichtung auf ein Absperrorgan einwirkt. Der Druck vor dem Filter kann nicht kontrolliert werden. Er ist normalerweise 50 bis 100 mmHg höher als der Druck hinter dem Filter. Dies ergibt sich aus der Flußgeschwindigkeit und dem Strömungswiderstand des Filters.

Während der Ansaugphase sind die Absperrorgange vor und hinter dem Filter geschlossen. Die Drücke zwischen diesen sind nicht mehr zu beeinflussen. Da kein Blutfluß mehr vorhanden ist, wird sich der Druck vor dem Filter dem Druck hinter dem Filter annähern. Beide Drücke werden zusätzlich absinken, weil Flüssigkeit vom Blut ins Dialysat wandert. Von konstanten Drücken kann man also nicht reden, erst recht nicht von konstantem Fluß.

Der Erfindung liegt die Aufgabe zugrunde, eine verbesserte, preiswerte Vorrichtung der gattungsgemäßen Art zu schaffen, die eine einfache Druckmessung sowie insbesondere eine Verbesserung der Einnadeldialyse ermöglicht.

Erfindungsgemäß wird die genannte Aufgabe bei einer Vorrichtung der eingangs genannten Art dadurch gelöst, daß als Zwischengefäß zumindestens ein flexibler Beutel zwischen zwei Andruckplatten angeordnet ist, von denen mindestens eine beweglich und antreibbar ist und daß der Beutel an einem Drucksensor anliegt.

Durch zumindestens eine auf dem Beutel wirkende bewegliche Platte kann die Drucküberwachung zur unmittelbaren Regelung und damit weitgehender Konstanthaltung desselben eingesetzt werden.

Erfindungsgemäß werden flexible Beutel, vorzugsweise Schlauchbeutel eingesetzt, die über Laschen in der Vorrichtung eingehängt werden können und mit einer Seite gegen einen Drucksensor drücken, der ggfls. an einer Platte angeordnet ist, während sie auf der dem Drucksensor abgewandten Seite mit einer Platte beaufschlagt werden, die dem Beutel gegen den Drucksensor hält, so daß hier eine zuverlässige Druckmessung vorgenommen werden kann. Es hat sich nämlich herausgestellt, daß bei einer derartigen Ausgestaltung in einem hinreichenden Seitenflächenbereich des Beutels ein repäsentativer Druckwert abgenommen werden kann. Ein bevorzugt zum Einsatz kommender Drucksensor ist dabei derart ausgestaltet, daß zwischen einer Platte und dem Drucksensor ein Übertrager angeordnet ist, wobei insbesondere der Übertrager eine Membran, gegen die der Beutel drückt und eine (nicht kompressible) Hydraulikflüssigkeit aufweist, die auf den Drucksensor drückt. Eine alternative Ausgestaltung sieht vor, daß der Drucksensor aus einer durch Schlitze in mehrere, lediglich im Randbereich miteinander verbundenen Teilflächen, geteilten Platten besteht, und daß zumindestens ein Teil der Teilflächen mit Dehnungsmeßstreifen versehen ist. Der Beutel kann vorzugsweise aus Weich-PVC bestehen. Eine der die Ausdehnung des Beutels seitlich begrenzenden Platten, vorzugsweise die dem Drucksensor gegenüberliegende ist vorzugsweise beweglich ausgebildet und in gewünschten Stellungen feststellbar. Sie kann daher zum Entnehmen des Beutels von diesem fortbewegt werden, so daß der Beutel entnommen und ausgetauscht werden kann. Andererseits kann sie in einer Stellung, indem sie den Beutel festhält und gegen den Drucksensor drückt, festgestellt werden. Die bewegliche Platte kann grundsätzlich linear verfahrbar sein. Damit die Platte nicht vollständig entfernt werden muß, ist sie vorzugsweise als um eine Achse verschwenkbare Klappe ausgebildet.

Die in der vorstehend erläuterten Weise ausgebildete erfindungsgemäße Vorrichtung läßt sich insbesondere mit erheblichem Vorteil bei der Einnadeldialyse einsetzen, indem die am Beutel anliegende Platte nicht nur beweglich, sondern durch einen Antrieb antreibbar ist, wobei eine Regelung des Antriebs derart erfolgt, daß der Druck im Beutel weitgehend konstant gehalten wird. Wie eingangs ausgeführt arbeiten sämtliche Einnadeldialysegeräte in zwei Phasen, wobei in einer ersten Phase mittels einer Pumpe das Blut aus dem Patienten abgezogen und in ein Zwischengefäß, wie ein Blasenfänger gedrückt wird. Dieses ist nach der erfindungsgemäßen Ausgestaltung der flexible Beutel, der gegen den Drucksensor drückt. Erhöht sich bei der konstanten Förderung durch die Pumpe der Druck über einen Sollwert, so wird die durch ihren Antrieb sich öffnende, und damit dem Beutel mehr Raum gebende Platte schneller bewegt und vice versa, so daß der Druck sich auf einen vorgegebenen Wert einstellen kann. In diesem Falle ist in üblicher bekannter Weise der venöse Zweig des Schlauchsystems der Vorrichtung gegenüber dem Patienten abgesperrt. Bei der zweiten Phase, bei der der venöse Zweig geöffnet und die Pumpe stillgesetzt ist, erfolgt das Zurückdrücken des Blutes in den Patienten durch die bewegliche Platte selbst, deren Geschwindigkeit derart druckgesteuert geregelt wird, daß ebenfalls nahezu ein konstanter Druck über die zweite Phase aufrechterhalten wird. Die Umschaltung zwischen beiden Phasen erfolgt vorzugsweise durch Weggeber oder Endschalter, die mit den beweglichen Platten zusammenwirken und von dieser zumindestens in ihren vorgesehenen und vorzugsweise einstellbaren Endstellungen beaufschlagt werden. Derart kann das Fördervolumen pro Phase in gewünschter Weise eingestellt werden. Während ein Zwischenbeutel oder Blasenfänger grundsätzlich vor oder hinter dem eigentlichen Dialysator angeordnet sein kann, sieht eine bevorzugte Ausgestaltung vor, daß sowohl vor als auch hinter dem Dialysatorfilter ein flexibler Beutel in der erfindungsgemäß ausgestalteten und angeordneten Weise vorgesehen ist und durch Platten bewegt wird, die einen gemeinsamen Antrieb aufweisen und gegebenenfalls einstückig ausgebildet sein können.

Während bei Einnadeldialysesystemen nach dem Stand der Technik erhebliche Druckschwankungen erfolgen können, auch innerhalb der jeweiligen Arbeitsphasen, stellt die Erfindung weitgehende Druckkonstanz sicher. Die erfindungsgemäße Vorrichtung bietet hierzu die Voraussetzungen, so daß ein Verfahren zur Durchführung einer Einnadeldialyse, wobei Blut von einem Patienten in einer ersten Phase unter Sperrung einer Rückflußleitung abgezogen und in einer zweiten Phase mit Unterbrechung der Absaugung und Öffnen der Rückflußleitung durch die gleiche Nadel wieder in die Blutbahn des Patienten zurückgedrückt wird, derart durchgeführt wird, daß das Blut unter Volumenveränderung von Zwischengefäßen nahezu in beiden Phasen druckkonstant gefördert wird. Hiermit sind insbesondere die Vorteile eines konstanten Transmembrandrucks bei einer Einnadeldialyse verbunden, wobei der venöse Abflußdruck wählbar ist und während der Rückflußphase im Gegensatz zum Stande der Technik konstant bleibt. Rückflußgeschwindigkeit und Rückflußzeit sind durch den Beuteldruck einstellbar, so daß sich ein weitgehend konstanter Blutdurchfluß im Dialysator erzielen läßt. Eine zweite Blutpumpe kann entfallen. Durch die flexiblen Beutel läßt sich ein geschlossenes System gegenüber der Atmosphäre schaffen und die Aufschäumung des Blutes, insbesondere durch kleine Luftblasen (Mikroschaum) wird vermindert, wodurch Gefahren reduziert werden können. Es lassen sich insbesondere stabilere Verhältnisse auch bei der Einnadeldialyse erzielen.

Wesentliche Vorteile der bevorzugten erfindungsgemäßen Ausgestaltung liegen also darin, daß der Druck in den Beuteln während beider Phasen mit Hilfe der Druckaufnehmer und der Druckplatten geregelt wird. Man kann den Druck vor dem Filter, nach dem Filter oder den Mittelwert konstant halten. Den Mittelwert konstant zu halten ist zweckmäßig, weil dadurch der Transmembrandruck konstant gehalten wird. Der Blutfluß im Filter und somit auch der Druckabfall am Filter sind ebenfalls konstant während beider Phasen, wobei diese die gleiche Dauer haben sollten. Dadurch sind Hin- und Rückfluß gleich. Der Filterfluß beträgt jeweils die Hälfte.

Insbesondere als Blasenfänger, aber auch zur direkten Druckmessung sind entsprechende Vorrichtungen mit einem flexiblen Beutel bekanntgeworden, bei denen allerdings Zu- und Abfluß diametral an zwei entgegengesetzten Enden des Beutel angeordnet sind. Hierdurch kann, wenn das Blut durch einen von oben in den Beutel hineinführenden Zufluß in diesen hereintropft, sich Schaum und Luftblasen bilden, was nachteilig ist. Auch kann aufgrund des freien Fallweges, wenn der Beutel nicht weitgehend gefüllt ist, das Blut sich im Beutel in nachteiliger Weise mit Luft ansammeln. In äußerst bevorzugter Ausgestaltung ist daher vorgesehen, daß der flexible Beutel mit einem Blutleitungssystem mit einem Zufluß- und Abflußschlauch am gleichen Ende in den Beutel münden. Durch diese Ausgestaltung, insbesondere die Anordnung der in den Beutel mündenden Enden von Zufluß- und Abflußleitung an einer gemeinsamen bzw. der gleichen Stirnseite des Beutels wird erreicht, daß, wenn der Beutel nicht weitgehend gefüllt ist, das Blut sehr schnell von der Zuflußleitung in die Abflußleitung gelangt und nicht durch einen größeren Luftraum hindurchtreten muß; insbesondere fällt das Blut nicht; es wird daher eine nachteilige Schaumbildung und eine mögliche Schädigung durch Hemolyse weitgehend verhindert. In weiterer bevorzugter Ausgestaltung ist vorgesehen, daß der Zuflußschlauch etwas weiter in den Beutel hineinragt als der Abflußschlauch. Hierdurch wird verhindert, daß, falls Luftblasen im Zulauf sind, die Möglichkeit besteht, daß diese in die Abflußleitung gelangen; sie werden vielmehr direkt im Beutel aufgefangen. Insbesondere wenn die Abflußleitung an der Unterkante des Beutels in diesem mündet, wird erreicht, daß der Beutel vollständig entleert werden kann. In an sich bekannter Weise kann weiterhin vorgesehen sein, daß an einer Seite, die der Seite, an der Zu- und Abflußschlauch in den Beutel münden, abgewandt ist, ein Be- und/oder Entlüftungsschlauch in das Beutelinnere mündet.

In weiterer Ausbildung kann vorgesehen sein, daß das Beutelinnere durch Querschweißnähte von sich jeweils jenseits anschließenden Befestigungslaschen abgegrenzt ist und insbesondere daß die Befestigungslaschen Aufhängelöcher aufweisen. Durch diese Ausgestaltung kann der Beutel in zuverlässiger und sicherer Weise befestigt, insbesondere aufgehängt werden.

Weitere Vorteile und Merkmale der Erfindung ergeben sich aus den Ansprüchen und aus der nachfolgenden Beschreibung, in der Ausführungsbeispiele unter Bezugnahme auf die Zeichnungen im einzelnen erläutert sind. Dabei zeigt:

Figur 1 Einen Flüssigkeitsaufnahmebeutel mit schematischer Darstellung des Drucksensors;

Figur 2 eine bevorzugte Ausführungsform eines Drucksensors;

Figur 3 eine grundsätzliche Anordnung einer erfindungsgemäßen Vorrichtung;

Figur 4 eine Seitenansicht der Anordnung der Figur 3 und

Figur 5 ein Blockschaltbild für die Einnadeldialyse.

Die Figur zeigt ein Behältnis in Form eines flexiblen Beutels 1, beispielsweise aus Weich-PVC. Der Beutel 1 ist aus einem Endlosschlauch hergestellt, bei dem mit Abstand zueinander zwei Querschweißnähte 2, 3 vorgesehen sind, zwischen denen der eigentliche Beutel gebildet ist. An die Querschweißnähte 2, 3 schließen sich Laschen 4, 6 zur Befestigung des Beutels in einem Dialysegerät an. Die Laschen weisen hierzu Querdurchbrüche oder Löcher 7, 8 auf. An der Unterseite des Beutels sind durch die Lasche 6 und die Naht 3 Zufluß- und Abflußleitungen hindurchgeführt, insbesondere durch in das Innere des Beutels 1 hineinragende Schlauchenden, durch die bei der Herstellung an ihren Durchtrittsstellen ein Verschweißen der Schweißnaht 3 verhindert wurde. In gleicher Weise kann im oberen Bereich des Beutels eine Leitung 13 zur Entlüftung und/oder Kontrolle des Flüssigkeitsspiegels vorgesehen sein.

Das innere Ende der Schlauchleitung 11 ragt im Inneren des Beutel etwas höher als das in diesen hineinragende Ende der Schlauchleitung 12, wobei letzteres möglichst nahe im Bodenbereich des Beutels, also nahe der Schweißnaht 3 angeordnet ist. Durch die Zuflußleitung 11 fließt Blut in den Beutel, beispielsweise unter Einwirkung einer Pumpe, ohne daß es über die gesamte Höhe des Beutels herunterfällt. Hierdurch wird eine Schaumbildung sowie eine Aufnahme von Luftblasen verhindert. Wenn die Abflußleitung 12 freigegeben ist, kann das Blut durch diese wieder aus dem Beutel herausfließen, sei es, daß es abgesaugt wird, sei es, daß der Beutel durch eine geeignete Vorrichtung zusammengedrückt wird, wodurch das Blut aus ihm herausgedrückt wird. Da die Mündung der Schlauchleitung 12 im Beutel in der Nähe der unteren Naht 3 angeordnet ist, kann das Blut nahezu vollständig aus den Beutel entfernt werden.

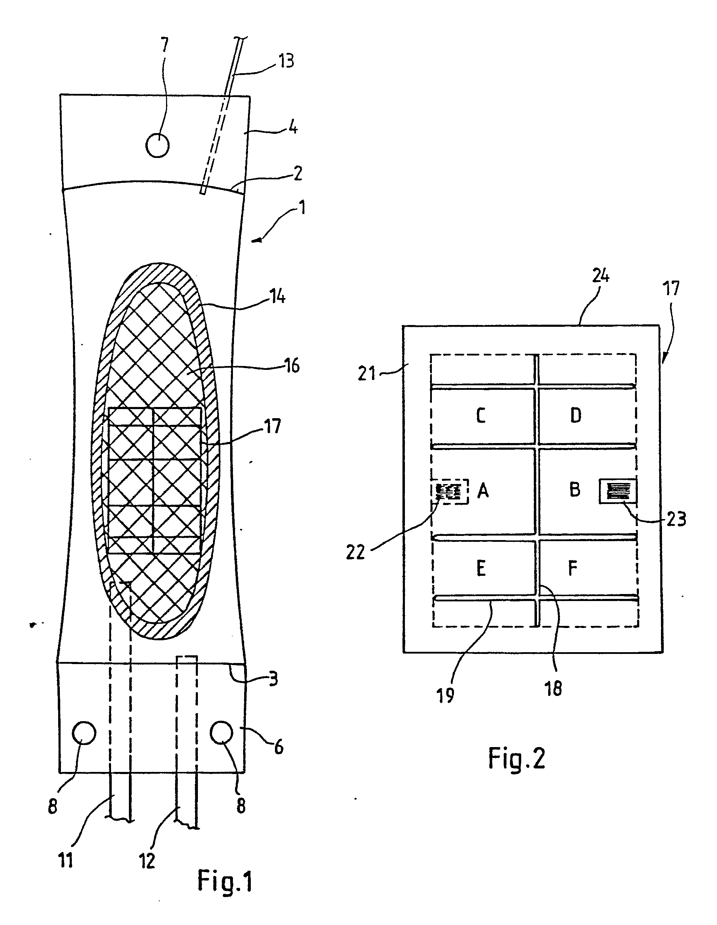

Bei der erfindungsgemäßen Vorrichtung liegt der Beutel im Einsatz - abhängig von seiner Füllmenge - etwa im schraffierten Bereich 14 an einer Klappe bzw. einer Platte der Dialysevorrichtung an, wobei im doppelt schraffierten Bereich 16 der Figur 1 ein nahezu konstanter Druck gegeben ist. In diesem Bereich weist die Klappe oder aber die Anlageplatte einen Drucksensor 17 auf, der im weiteren näher beschrieben ist.

Der Drucksensor 17 besteht im dargestellten Ausführungsbeispiel aus einem mit Längs- und Querschlitzen 18,19 versehenen Blech, vorzugsweise aus Stahl. Durch die Schlitze 18,19 werden mehrere Teilflächen A bis F gebildet, die über den Rand 21 miteinander verbunden sind. Die beiden mittleren Teilflächen A,B sind in ihrem Randbereich mit Dehnungsmeßstreifen 22,23 versehen, die über Leitungen mit einem Verstärker verbunden werden und an die jeweils Spannung angelegt wird. Wenn die Flächen A,B aus der Ebene des Stahlblechs 17 herausgebogen wurden, ändern die Dehnungsmeßstreifen ihren Widerstand. Das Stahlblech 17 kann in einen Rahmen 24, beispielsweise aus Messing eingesetzt sein, der dem Stahlblech 17 im Randbereich 21 eine hinreichende Stabilität und Biegesteifigkeit verleiht.

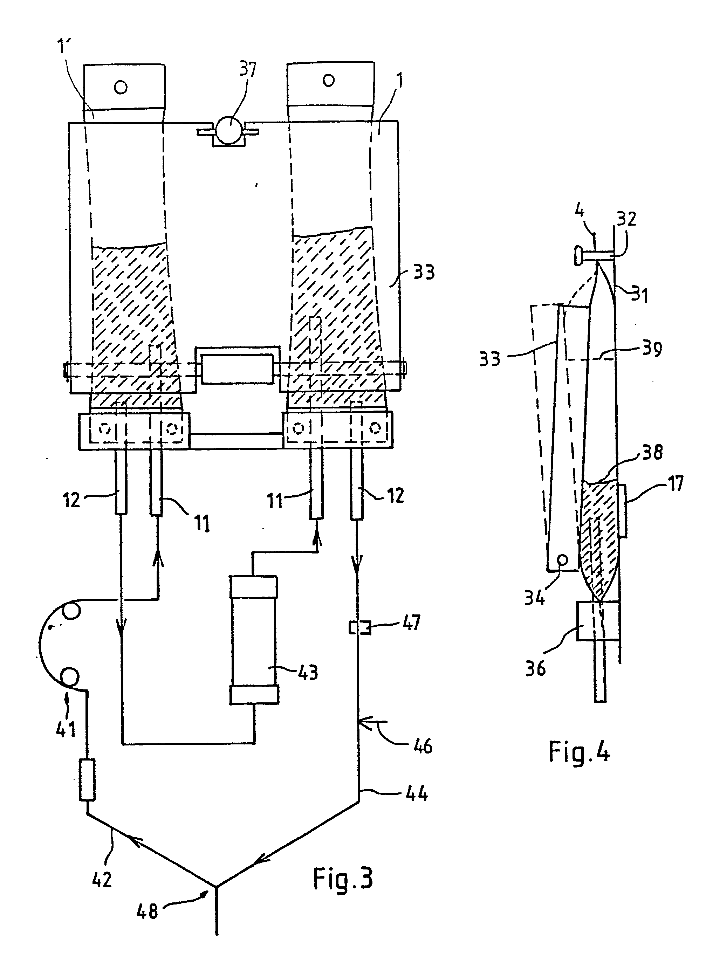

Wenn der Drucksensor, wie in den Figuren 1 und 4 dargestellt, am Beutel 1 anliegt, so werden die Felder A bis F in Abhängigkeit vom Druck im Beutel 1 ausgelenkt, die auf den Feldern A und B befindlichen Dehnungsmeßstreifen ändern ihren Widerstand, so daß über den Verstärker, an dem sie angeschlossen sind, ein Signal in Abhängigkeit vom Druck im Beutel abgegriffen und zur Weiterverarbeitung in der unten beschriebenen Weise bzw. zur Überwachung abgegriffen werden kann. Die Felder C bis F sind lediglich Ausgleichsflächen, die mit den eigentlichen Meßfeldern A und B einfedern. Hierdurch wird eine Stufenbildung am Rande der Flächen A und B verhindert, die zu einem Meßfehler der Messung über die Felder A und B führen könnten, welcher durch die dargestellte bevorzugte Ausgestaltung mit den zusätzlichen Ausgleichfeldern C bis F vermieden wird.

Ein Beutel 1 wird als Blasenfänger an einer Halterungsplatte 31, die einen Halterungsstift 32 aufweist mit dem Loch 7 in der Lasche 4 aufgehängt und liegt dabei mit seiner einen Seite an der Halterungsplatte 31 an. Diese weist einen Durchbruch auf, in dem der Drucksensor 17 derart angeordnet ist, daß die Beutelfläche in diesem Bereich in der beschriebenen Weise gegen die Felder A bis F (Fig.2) des Drucksensors drücken kann. Mit Abstand zur Halterungsplatte 31 ist eine Platte 33 vorgesehen, die um eine relativ zur Platte 31 feste horizontale Achse 34 um einen geringen Winkelbereich schwenkbar ist. In der Fig.4 ist in durchgezeichneten Linien die Stellung bei minimalem Beutelinhalt und gestrichelt die Stellung bei maximalem Beutelinhalt dargestellt. Die Stellung der Platte 33 kann durch einen Wegaufnehmer, beispielsweise in Form eines Potentiometers gemessen werden, wobei weiterhin Endschalter vorgesehen sein können (nicht dargestellt). Im unteren Bereich wird der Beutel durch eine weitere Halterung 36 gehalten, die den Zufluß 11 und Abfluß 12 übergreift. Die Bewegung der Platte 33 erfolgt beispielsweise über eine Schub- und Zugstange 37, wie sie in der Fig.3 angedeutet ist. Die Platte 33 besteht vorzugsweise aus Transparentmaterial, wie Acrylglas, damit der Flüssigkeitsstand 38 (minimaler Flüssigkeitsstand) bzw. 39 (maximaler Flüssigkeitsstand) beobachtet werden kann. Im dargestellten Ausführungsbeispiel der Fig.3 sind zwei Gefäße an einer gemeinsamen Platte 31, auf die eine gemeinsame Platte 33 wirkt, vorgesehen.

Der Zufluß 11 des ersten Beutels 1'ist über eine Zuführ-Blutpumpe 41 mit einem Gefäß des Patienten über einen Anschluß 42 verbunden. Das Gefäß ist im Zweinadelbetrieb regelmäßig die Arterie, während es im Einnadelbetrieb in der Regel eine Vene ist, die selbst mit einer Arterie verbunden ist. Der Auslaß 12 des hierdurch gegebenen arteriellen Beutels 1'ist mit dem eigentlichen Dialysator 43 verbunden, der in bekannter Weise die Filtermembran aufweist, über die hin die Dialyse erfolgt. Der Dialysator 43 ist weiterhin mit dem Einlaß 11 des venösen Beutels 1'verbunden, dessen Auslaß 12 über eine Leitung 44 mit einer Vene des Patienten in Verbindung steht. Die Auslaßleitung 43 weist eine Schlauchklemme 46 auf, die durch einen auf die Leitung 44 wirkenden Pfeil angedeutet ist. Weiterhin kann ein Luftfallensensor 47 vorgesehen sein.

Die Pumpe 41 ist eine herkömmliche Blutpumpe, die im Betrieb (quasi-) kontinuierlich Blut fördert. Sie weist in üblicher Ausgestaltung eine drehbare Welle auf, an der ein Bügel angebracht ist, der an seinem Ende drehbare Zylinder trägt. Etwa über die Hälfte des Kreisbogens, der von den Zylindern durchfahren wird, erstreckt sich eine halbkreisförmige Führungsbahn. Die Arterienschlauchleitung folgt der inneren Oberfläche der Führungsbahn. Auf diese Weise wird dann, wenn die Zylinder ihre Kreiswege um die Achse der Welle durchfahren, die Arterienschlauchleitung zwischen einem Zylinder und der Führungsbahn zusammengedrückt und durch die Bewegung des Zylinders mit diesem über die Führungsbahn hinwegwandern, wodurch das Blut durch die Zylinder in deren Bewegungsrichtung gefördert wird.

Beim Einnadelbetrieb sind Zuflußleitung 42 und Rückflußleitung 44 miteinander über ein T-Stück 48 verbunden, wie dies in der Fig.3 dargestellt ist, während beim Zweinadelbetrieb, wie gesagt, beide Leitungen 42,44 separat mit den entsprechenden Gefäßen des Patienten verbunden sind.

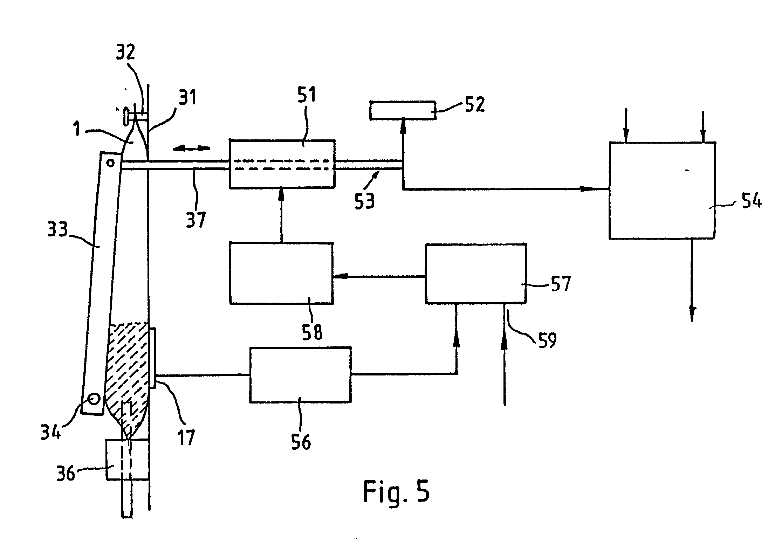

Blockbildartig ist die Schaltung zur Steuerung des Ablaufs in der Fig.5 dargestellt. Die Schub- und Zugstange 37 (Fig. 3) wird durch einen Antrieb 51 bewegt und ist, wie gesagt, an der Platte 33 angelenkt, um diese zu verstellen. Der Antrieb kann ein Spindel- oder Zahnstangenantrieb sein. Zur Bestimmung der Position der Platte 33 ist ein Weggeber 52 wie ein Potentiometer vorgesehen, auf den das der Platte 33 abgewandte Ende der Zug- und Schubstange 37 wirkt. Endpositionen der Stange 37 werden weiterhin durch einen Schwellwertschalter 54 erfaßt, der die Antriebsrichtung des Antriebs 51 umschaltet und damit dem Einnadelbetrieb einen Wechsel der Arbeitsphasen schaltet (hierauf wird weiter unten eingegangen). Dem Sensor 17 ist der schon erwähnte Verstärker 56 für das Drucksensorsignal zugeordnet, dem weiterhin ein Soll-Istwert-Vergleicher 57 nachgeordnet ist, der die Leistungsstufe 58 des Antriebs 51 regelt. Da die erfindungsgemäße Vorrichtung mit konstantem Druck im Beutel 1 arbeiten soll, wird über den Soll-Istwert-Vergleicher über dessen Druck-Sollwert-Eingang 59 ein Drucksollwert vorgegeben, beispielsweise eingestellt und im Vergleicher 57 mit dem Druck-Istwert vom Sensor 17 verglichen. Durch den Vergleicher wird die Leistungsstufe 58 des Antriebs 51 derart eingeregelt, daß der Druck-Istwert weitgehend konstant im Bereich des DruckSollwerts gehalten wird. Wenn beim Ausfüllen der Beutel der Druck-Istwert den Sollwert aufgrund der Leistung der Pumpe 41 überschreitet, wird die Öffnung der Platte 33 beschleunigt, wenn bei dieser Phase der Sollwert unterschritten wird, verlangsamt, um den Gegendruck zu erhöhen. In der zweiten Phase der Einnadeldialyse beim Zurückdrücken der in den Beuteln befindliche Flüssigkeit in die Blutbahn des Patienten wird bei Unterschreiten des Ist-Sollwerts die Bewegungsgeschwindigkeit der Platte 33 erhöht, um die Flüssigkeit mit größerer Geschwindigkeit aus dem Beutel zu drücken und damit auch den Druck zu erhöhen und vice versa.

Der Ablauf der Einnadeldialyse ist derart, daß zunächst in einer ersten Phase die Schlauchklemme 46 geschlossen ist und Blut aus der Blutbahn des Patienten mit der Pumpe 41 in den Beutel 1'-(Fig.3) und durch diesen und den Filter 43 in den Beutel 1 unter Kostanthaltung des Drucks mittels der vorstehend beschriebenen Regeleinrichtung gedrückt wird. Bei Erreichen der maximalen Öffnungsstellung der Platte 33 erfolgt über den Schwellwertschalter 54 ein Abschalten der Pumpe 41 ein Umschalten des Antriebs 51 sowie ein Öffnen der Schlauchklemme 46, so daß in der nächsten Phase das in den Beuteln 1,1'befindliche Blut durch die Klappe 33 aus diesen heraus und über die Rückflußleitung 44 zum Patienten zurückgedrückt wird. Bei Erreichen der Minimalstellung der Platte 33 erfolgt wieder ein Schaltvorgang, der die Pumpe 41 einschaltet, die Schlauchklemme 46 schließt und den Antrieb 51 umschaltet.

Bei der Zweinadeldialyse kann grundsätzlich die gleiche Vorrichtung eingesetzt werden, da in diesem Falle ein kontinuierlicher Durchfluß des Blutes durch die Dialysevorrichtung erfolgen kann, wird die Platte 33 während des Betriebs lediglich druckgeregelt um Druckschwankungen auszugleichen. Zum Auswechseln der Beutel kann sie in ihre maximale Öffnungsstellung verfahren werden, um das Auswechseln zu vereinfachen.

## Ansprüche

1. Vorrichtung zur Dialyse, insbesondere zur Einnadel-Dialyse, mit einem Dialysator, mindestens einer Blutpumpe, Zwischengefäßen und Drucksensoren, dadurch gekennzeichnet, daß als Zwischengefäß zumindestens ein flexibler Beutel (1) zwischen zwei Andruckplatten (31,33) angeordnet ist, von denen mindestens eine beweglich und antreibbar ist und daß der Beutel an einem Drucksensor (17) anliegt.

2. Vorrichtung nach Anspruch 1, dadurch gekennzeichnet, daß die bewegliche Platte (33) druckgeregelt antreibbar ist.

3. Vorrichtung nach Anspruch 1 oder 2, dadurch gekennzeichnet, daß der Drucksensor (17) über einen Vergleicher (57) regelnd mit einer elektrischen Antriebsstufe (58) für den Antrieb der beweglichen Platte (33) verbunden ist.

4. Vorrichtung nach einem der Ansprüche 1 bis 3, dadurch gekennzeichnet, daß die bewegliche Platte (33) während einer Pumpphase der Blutpumpe (41) frei beweglich und während einer Ruhephase der Blutpumpe (41) gegen die weitere Platte (31) drückbar ist.

5. Vorrichtung nach einem der vorangehenden Ansprüche, dadurch gekennzeichnet, daß ein Beutel (1) im Blutkreislauf vor dem Dialysator (43) und ein weiterer Beutel (1) im Blutkreislauf hinter dem Dialysator angeordnet sind.

6. Vorrichtung nach Anspruch 5, dadurch gekennzeichnet, daß die beweglichen Platten (33) der Beutel synchron antreibbar sind.

7. Vorrichtung nach Anspruch 5 oder 6, dadurch gekennzeichnet, daß die Beutel (1,1') durch eine gemeinsame Platte beaufschlagt werden.

8. Vorrichtung nach einem der Ansprüche 1 bis 7, dadurch gekennzeichnet, daß ein Übertrager zwischen einer Platte (31) und dem Drucksensor (17) eine Membran, gegen die der Beutel (1) drückt, und eine (nicht kompressible) Hydraulikflüssigkeit aufweist, die auf den Drucksensor drückt.

9. Vorrichtung nach einem der vorangehenden Ansprüche, dadurch gekennzeichnet, daß der Drucksensor (17) aus einer durch Schlitze in mehrere, lediglich im Randbereich miteinander verbundenen Teilflächen geteilten Platte besteht und daß zumindestens ein Teil der Teilflächen (A bis F) mit Dehnungsmeßstreifen versehen ist.

10. Vorrichtung nach einem der Ansprüche 1 bis 9, dadurch gekennzeichnet, daß der beweglichen Platte (33) ein Weggeber zugeordnet ist.

11. Vorrichtung nach einem der Ansprüche 1 oder 10, dadurch gekennzeichnet, daß mit der beweglichen Platte ein Weggeber (52) und/oder Schwellwertschalter (54) mit einer Einrichtung zum Ein-/ Ausschalten der Pumpe (41), zum Umschalten des Antriebs (51) für die Platte (31) sowie einer Sperreinrichtung der Leitungen verbunden sind.

12. Vorrichtung nach einem der Ansprüche 1 bis .11, dadurch gekennzeichnet, daß der flexible Beutel (1) mit einem Blutleitungssystem mit einem Zufluß- (11) und einem Abflußschlauch (12) versehen ist und daß Zufluß- und Abflußschlauch (11, 12) am gleichen Ende (bei 3) in den Beutel (1) münden.

13. Vorrichtung nach Anspruch 12, dadurch gekennzeichnet, daß der Zuflußschlauch (11) zumindestens etwas weiter in dem Beutel (1) hineinragt als der Abflußschlauch (12).

14. Vorrichtung nach Anspruch 12 oder 13, dadurch gekennzeichnet, daß an einer Seite (bei 2), die der Seite, an der Zu- und Abflußschlauch (11, 12) in den Beutel (1) münden, abgewandt ist, ein Be-und/oder Entlüftungsschlauch (13) in das Beutelinnere mündet.

15. Vorrichtung nach einem der vorangehenden Ansprüche, dadurch gekennzeichnet, daß das Beutelinnere durch Querschweißnähte (2, 3) von sich jeweils jenseits anschließende Befestigungslaschen, (4, 6) abgegrenzt ist.

16. Verfahren zur Durchführung einer Dialyse, wobei der Druck im Blutkreislauf zumindestens hinter dem Dialysator gemessen wird, dadurch gekennzeichnet, daß der Druck konstant geregelt wird.

17. Verfahren nach Anspruch 16, insbesondere zur Einnadel-Dialyse, wobei Blut von einem Patienten in einer ersten Phase unter Sperrung einer Rückflußleitung abgezogen und in einer zweiter Phase mit Unterbrechung der Absaugung und Öffnen der Rückflußleitung durch die gleiche Nadel wieder in die Blutbahn des Patienten zurückgedrängt wird, dadurch gekennzeichnet, daß das Blut unter Volumenveränderung von Zwischengefäßen nahezu in beiden Phasen druckkonstant gefördert wird.

18. Verfahren nach Anspruch 17, dadurch gekennzeichnet, daß in der zweiten Phase das Blut dadurch gefördert wird, daß zumindestens eine Platte wenigstens einen als Zwischengefäß dienenden flexiblen Beutel aktiv zusammendrückt.

19. Verfahren nach einem der vorangehenden Ansprüche, dadurch gekennzeichnet, daß während der ersten Phase des Abzugs vom Blut aus der Blutbahn des Patienten das Blut teilweise in ein erstes, vor dem Dialysator angeordnetes Zwischengefäß und teilweise durch den Dialysator hindurch in das zweite Zwischengefäß gepumpt wird, während in der zweiten Phase das Blut aus dem ersten Zwischengefäß durch den Dialysator hindurch zusammen mit dem Blut aus dem zweiten Zwischengefäß in die Blutbahn zurückgefördert wird.

20. Verfahren nach Anspruch 19, dadurch gekennzeichnet, daß das Blut in der zweiten Phase durch aktive Volumenänderung der Zwischengefäße gefördert wird.

Fig.1

Fig.2

Fig.3

Fig.4

Fig. 5

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | DE - A1 - 3 720 664 (SCHAL) * Gesamt; besonders Spalte 7, Zeile 63 - Spalte 8, Zeile 6; Spalte 8, Zeilen 10-52; Spalte 11, Zeile 41 - Spalte 12, Zeile 12; Figuren 2-5 * -- | 1,5, 12,14 | A 61 M 1/14 A 61 M 1/30 |
| A | US - A - 4 643 714 (BROSE) * Spalte 2, Zeilen 33-62; Fig. 1 * -- | 1,12, 13 | |
| A | US - A - 4 270 533 (ANDREAS) * Zusammenfassung, Zeilen 1-4; Spalte 3, Zeilen 49-62; Spalte 4, Zeilen 27-36; Fig. 2,3a,3b * ---- | 12,15 | |

**RECHERCHIERTE SACHGEBIETE (Int Cl⁵)**

A 61 M 1/00

Der vorliegende Recherchenbericht wurde für alle Patentansprüche erstellt.

| Recherchenort WIEN | Abschlußdatum der Recherche 28-05-1990 | Prüfer VELINSKY-HUBER |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82